# EUROPEAN PATENT APPLICATION

(11) **EP 4 582 443 A1**
(43) Date of publication of application: **09.07.2025**
(21) Application number: 22956977.7
(22) Date of filing: 02.09.2022
(51) Int. Cl.: C07K 14/765, C12N 15/81, C12N 15/14, C12N 1/19, C12N 15/53, C12R 1/78, C12R 1/72, C12R 1/84

(54) **METHOD FOR IMPROVING EXPRESSION LEVEL OF RECOMBINANT HUMAN ALBUMIN, AND CELL AND PROTEIN**

(71) Applicant: Tonghua Anrate Biopharmaceutical Co., Ltd., Tonghua, Jilin 134100 (CN)
(72) Inventor: XIANG, Wei, Tonghua, Jilin 134100 (CN); HAN, Xu, Tonghua, Jilin 134100 (CN)
(74) Representative: Winter, Brandl - Partnerschaft mbB
(86) International application number: PCT/CN2022/116713
(87) International publication number: WO 2024/045153

(57) **Abstract**

The invention discloses a recombinant human albumin expression level improving method and cells and proteins, and it belongs to the field of biopharmaceutical technologies. While expressing recombinant human albumin, the invention weakens, damages or removes the functions of the endogenous genes of at least one encoding flocculating protein, and/or edits the endogenous genes or the heterologous genes to allow the over-expression of the superoxide dismutase; and/or edits the endogenous genes or the heterologous genes to allow the over expression of the E3 ubiquitin ligase. As a result, the expression of the recombinant human albumin is improved.

## Description

### Field of Technology

This invention involves a recombinant human albumin expression level improving method, and it specifically involves a recombinant human albumin expression level improving method and cells and proteins.

### Background

Pichia Pasteuris is genetically stable, with strong and strictly regulated promoters, and an intracellular environment and a sugar chain processing system suitable for the correct folding of eukaryotic gene products. It is a kind of eukaryotic expression system for protein production that is simple in operations, can be used for large scale high density culturing and fermentation, and is widely used in recent years.

Yeast has two kinds of propagation, vegetative propagation and generative propagation. During the vegetative propagation of the yeast, when there is any external environmental stress, it will lead to the flocculation of the yeast, and the death of the yeast can be resulted when the environmental stress is high. The generative propagation, on the other hand, is the mating of two adjacent yeast of different sexes. The fusion of haploid spores produces diploids. And in harsh conditions, the reduction meiosis produces haploid spores.

Yeast flocculation is a vegetative, reversible and calcium-dependent process, which is a kind of multicellular aggregation resulted from the binding of the flocculating proteins encoded by flocculating genes and the mannose on the adjacent cell walls. The cells will precipitate from the fermentation broth after premature flocculation, which may lead to incomplete fermentation. It weakens, damages or removes the functions of the endogenous genes of at least one encoding flocculating protein, including FLO5, FLO8 and FLO11, thus inhibiting the formation of pseudohypha. Add mannose into the culture medium, allow it to occupy the binding site of the flocculating protein the binding to the mannose residues on the adjacent cell walls is prohibited, and the flocculation is thus inhibited.

The REDOX state in yeast cells directly affects the survival, activation and proliferation of the cells. When methanol is used as the carbon source, methanol is decomposed into formaldehyde and hydrogen peroxide with the help of alcohol oxidase, and a large number of reactive oxygen species (ROS) are generated during the metabolic process of hydrogen peroxide, which can lead to direct damage to such cell components as nucleic acids, lipids and proteins. In severe cases, it can also cause cell senescence or death. Superoxide dismutase catalyzes the formation of hydrogen peroxide and oxygen by superoxide anion radicals. It can effectively resist the toxicity of superoxide ions to cells, protect the body from oxidative damages, maintain the body balance, and extend the cell life.

In a yeast cell, the endoplasmic reticulum is an important place for the synthesis and the maturation of secretory proteins. It is rich in various molecular chaperones and folding enzymes that assist in protein folding and modification, such as protein disulfide isomerase (PDI). PDI can catalyze the formation and the rearrangement of the disulfide bonds between cysteine residues, help the correct folding of proteins, and improve their expression.

Human Serum Albumin (HSA) is a kind of single chain spherical protein composed of 585 amino acids. When proteins are blocked at the disulfide bond formation phase or the transportation from the endoplasmic reticulum to the Golgi apparatus, the unfolded or misfolded proteins gather in the endoplasmic reticulum, causing a pressure in the endoplasmic reticulum and thus affecting the normal cell functions. There are two major pathways for the regulation of the endoplasmic reticulum pressure, namely the unfolded protein response (UPR) and the endoplasmic reticulum associated degradation (ERAD). The UPR improves the folding ability of proteins by up-regulating the expression levels of the folding enzymes and the molecular chaperones through a series of signal transduction responses in the cells. The ERAD transports the protein substrates that cannot be correctly folded from the endoplasmic reticulum to the cytoplasm through reverse transportation, where they are further degraded by the proteasome through ubiquitination labeling.

The ubiquitin proteasome pathway consists of ubiquitin and a series of related enzymes. Ubiquitin activating enzyme E1 relies on the energy supplied by ATP to activate ubiquitin molecules and transfer them to ubiquitin binding enzyme E2. Ubiquitin ligase E3 recognizes the target protein to be degraded and connects the ubiquitin binding enzyme E2 to the Lys residue of the target protein. This process is repeated so that the target protein binds to multiple ubiquitin. With the help of 26S proteasome, it is degraded into small peptides composed of amino acid residues. The proteasome itself is not selective for proteins, it is the E3 ubiquitin ligase that has the selective specificity.

### Definitions and Terminologies

"Host cell" means a cell that receives a foreign gene during the transformation or transduction process.

"Expression cassette" means a gene expression system containing all the necessary elements required for the expression of the foreign proteins, including promoters, cloning sites of foreign genes, signal peptide sequences, mature peptide coding sequences of target proteins, terminators, screening markers, etc.

"Vector" means the autonomous DNA that can carry foreign DNA into the host cell for replication or ultimately for the expression of foreign DNA. They are mainly divided into cloning vectors and expression vectors. The former vectors are mainly used for gene replication and amplification, and the latter vectors are mainly used for the expression of target genes.

"DNA" means deoxyribonucleic acid.

"Gene expression" means the process in which the genetic information carried by the structural genes in the biological genome is used through a series of processes such as transcription and translation to synthesize a specific protein and further exerts its specific biological functions.

"Operable linkage" means the covalently bonded linkage of the regulatory elements to the coding sequence through transcription and translation in such a spatial arrangement that the regulatory elements can direct the expression of the coding sequence.

"Signal peptide" means the N-terminal amino acid sequence in a newly synthesized polypeptide chain that guides the transmembrane transfer of the protein.

"Molecular chaperone" means the proteins and peptides that help the macromolecular structures of the cells fold correctly.

"Recombinant promoter" means a genetically modified or unmodified promoter that is not naturally present at the upstream side of a gene in a genome or a wild-type promoter. It is a specific DNA sequence that exists upstream of the 5' terminal of the coding sequence of the target gene and identifies and binds to RNA polymerase to control the transcription of the target gene.

"FDH" Formate dehydrogenase,

"FLD" Formaldehyde dehydrogenase,

"GAL" Galactose,

"GAP" Glyceraldehyde-3-phosphate dehydrogenase,

"UPR" unfolded protein response,

"ERAD" ER associated degradation,

"ROS" Reactive Oxygen Species,

"HSA" Human Serum Albumin,

"PDI" Protein Disulfide Isomerase,

"LB" Luria bertani medium,

"MD" Minimal Dextrose medium,

"BMGY" Buffered Glycerol-complex Medium,

"BMMY" Buffered Methanol-complex Mdeium,

"YPD" Yeast extract/peptone/dextrose-media,

"Zeo" zeocin,

"His" Histidinol dehydrogenase,

"Da" Dalton,

"ml" milliliter,

"Mut^{s}" Methanol utilization slow,

"Mut⁺" Methanol utilization plus,

"PCR" Polymerase chain reaction,

"rpm" Rounds per minute,

"SDS-PAGE" Sodium dodecyl sulphate polyacrylamide gel electrophoresis,

"SEQ ID NO." Sequence Identity Document Number,

### Invention Description

Invention Purpose: The purpose is to provide a more effective recombinant human albumin expression level improving method and cells and proteins. Please see the multiple substantive technical effects of the specific embodiments for the specific purpose.

In order to achieve the above purpose, the invention adopts the following technical embodiments.

### Embodiment I:

A method for improving the expression of recombinant human albumin, which is characterized by that, the recombinant host cell weakens, damages or removes the functions of the endogenous genes of at least one encoding flocculating protein, and/or edits the endogenous genes or the heterologous genes to allow the over-expression of the superoxide dismutase; and/or edits the endogenous genes or the heterologous genes to allow the overexpression of the E3 ubiquitin ligase.

### Embodiment II:

A method for improving the expression of recombinant human albumin, where the embodiment steps include,
(1) The host cell that encodes at least one recombinant human albumin;
(2) The host cell that weakens, damages or removes the functions of the endogenous genes of at least one encoding flocculating protein;
(3) The host cell that encodes at least one endogenous or heterologous superoxide dismutase;
(4) The host cell that encodes at least one endogenous or heterologous E3 ubiquitin ligase.

A further technical embodiment of the invention is that the host cell is a yeast cell.

A further technical embodiment of the invention is that the yeast cell is the cell of one or more of the following yeasts: Hansenula, Pichia and Candida.

A further technical embodiment of the invention is that the Pichia cell is Pichia pastoris cell.

A further technical embodiment of the invention is that the host cell has weakened, damaged or removed the functions of the endogenous genes of at least one encoding flocculating protein, and the endogenous genes of the flocculating protein include FLO5, FLO8 and FLO11.

A further technical embodiment of the invention is that the gene encoding superoxide dismutase is genetically modified or not genetically modified.

A further technical embodiment of the invention is that the gene encoding superoxide dismutase is yeast superoxide dismutase, and more preferred human copper-zinc superoxide dismutase nucleic acid molecule.

A further technical embodiment of the invention is that the gene encoding E3 ubiquitin ligase is genetically modified or not genetically modified.

A further technical embodiment of the invention is that the gene encoding E3 ubiquitin ligase is yeast E3 ubiquitin ligase, and more preferred human E3 ubiquitin ligase nucleic acid molecule.

A further technical embodiment of the invention is that there is at least one recombinant promoter modified human albumin encoded in the host cell.

A further technical embodiment of the invention is that the recombinant expression cassette of the gene encoding human albumin, the gene encoding superoxide dismutase, and the gene encoding E3 ubiquitin ligase is based on a nucleic acid construct or the nucleic acid construct of different selection markers.

A further technical embodiment of the invention is that the nucleic acid constructs include but are not limited to the plasmids pHIL-D2, pPIC3.5, pHIL-S1, pPIC9, pPink-LC, pPink-HC, pPICZA, pPICZB, pPICZC, pPICZaA, pPICZaB and pPICZaC.

A further technical embodiment of the invention is that the recombinant promoters include but are not limited to AOX1 promoter, GAP promoter, GAL promoter, FDH promoter and FLD promoter.

A further technical embodiment of the invention is that, a method for producing recombinant proteins is provided, where the host cell has weakened, damaged or removed the functions of the endogenous genes of at least one encoding flocculating protein, and the endogenous genes of the flocculating protein include FLO5, FLO8 and FLO11; encodes at least one recombinant promoter modified human albumin; and the host cells are cultured at conditions suitable for the production of recombinant proteins.

A further technical embodiment of the invention is that, a method for producing recombinant proteins is provided, where the host cell has weakened, damaged or removed the functions of the endogenous genes of at least one encoding flocculating protein, and the endogenous genes of the flocculating protein include FLO5, FLO8 and FLO11; encodes at least one endogenous or heterologous superoxide dismutase; encodes at least one recombinant promoter modified human albumin; and the host cells are cultured at conditions suitable for the production of recombinant proteins.

A further technical embodiment of the invention is that, a method for producing recombinant proteins is provided, where the host cell has weakened, damaged or removed the functions of the endogenous genes of at least one encoding flocculating protein, and the endogenous genes of the flocculating protein include FLO5, FLO8 and FLO11; encodes at least one endogenous or heterologous superoxide dismutase; encodes at least one endogenous or heterologous E3 ubiquitin ligase; encodes at least one recombinant promoter modified human albumin; and the host cells are cultured at conditions suitable for the production of recombinant proteins.

A method for improving the expression of recombinant human albumin, which is characterized by that the sequence used is any or any combination of the following sequences:
SEQ ID NO.1 encoding sequence of mating factor signal peptide of Saccharomyces cerevisiae;
SEQ ID NO.2 encoding sequence of signal peptide of human albumin;
SEQ ID NO.3 encoding sequence of mature peptide of human albumin;
SEQ ID NO.4 encoding sequence of yeast superoxide dismutase;
SEQ ID NO.5 encoding sequence of human superoxide dismutase;
SEQ ID NO.6 encoding sequence of yeast E3 ubiquitin;
SEQ ID NO.7 encoding sequence of yeast flocculating protein FLO5;
SEQ ID NO.8 encoding sequence of yeast flocculating protein FLO8;
SEQ ID NO.9 encoding sequence of yeast flocculating protein FLO11;
SEQ ID NO.10 HSA-F primer sequence;
SEQ ID NO.11 HSA-R primer sequence;
SEQ ID NO.12 sequence of yeast superoxide dismutase-F;
SEQ ID NO.13 sequence of yeast superoxide dismutase-R;
SEQ ID NO.14 sequence of human superoxide dismutase-F;
SEQ ID NO.15 sequence of human superoxide dismutase-R;
SEQ ID NO.16 encoding sequence of yeast E3 ubiquitin-F;
SEQ ID NO.17 encoding sequence of yeast E3 ubiquitin-R;

The host cells manipulated by any or any combination of the sequence genes SEQ ID NO.1 - SEQ ID NO.17.

The recombinant albumins expressed by host cells manipulated by any or any combination of the sequence genes SEQ ID NO.1 - SEQ ID NO.17.

This invention adopting the above mentioned technical embodiments has the following beneficial effects compared with the prior art. This invention can maximize the expression level of recombinant human albumin. This invention provides a recombinant human albumin expression level improving method by weakening, damaging or removing the functions of the endogenous genes of at least one encoding flocculating protein, and/or editing the endogenous genes or the heterologous genes to allow the over-expression of the superoxide dismutase; and/or editing the endogenous genes or the heterologous genes to allow the overexpression of the E3 ubiquitin ligase.

### Drawing Descriptions

In order to further explain this invention, it is further explained in conjunction with the attached figures:
Figure 1 shows the structure of the recombinant expression cassette encoding human albumin.
Figure 2 shows the structure of the recombinant expression cassette encoding E3 ubiquitin ligase.
Figure 3 shows the structure of the recombinant expression cassette encoding superoxide dismutase.
Figure 4 shows the structure of the recombinant expression cassette encoding superoxide dismutase and E3 ubiquitin ligase.
Figure 5 shows the structure of the recombinant expression cassette of the FLO gene knockout plasmid.
Figure 6 shows the SDS-PAGE result of the recombinant human albumin expression in embodiment 3. Lane 1 shows the recombinant engineering strain IV, lane 2 shows the recombinant engineering strain V, lane 3 shows the recombinant engineering strain VI, lane 4 shows the recombinant engineering strain I, lane 5 shows the recombinant engineering strain II, and lane 6 shows the recombinant engineering strain III. The recombinant human albumin expression level is improved significantly.
Figure 7 shows the relative expression levels of recombinant human albumin of each recombinant engineering strains in Embodiment 3.

### Actual Embodiments

This patent provides multiple parallel embodiments. The different expressions are for the improved embodiments based on the basic embodiment or parallel embodiments. Each embodiment has its own unique characteristics.

The purpose of this invention is to provide a method for improving the expression of recombinant human albumin.

Therefore, this invention involves the methods for improving the expression of recombinant human albumin, including but not limited to:

### 1) Genetically modified yeast cells

The recombinant promoters of the said yeast cells include but are not limited to AOX1 promoter, GAP promoter, GAL promoter, FDH promoter and FLD promoter.

The yeast cells include but are not limited to Hansenula, Pichia, Schizosacchromyces, Candida, Schizosaccharomyces, Torulopsis and Aspergillus. Pichia is preferred and Pichia pastoris is more preferred.

The phenotypes of the yeast cells are preferred to be methanol utilization slow (Mut^{s}), including but not limited to KM71 and KM71H, or the methanol utilization plus (Mut+), including but not limited to GS115, X-33 and CBS7435.

The said yeast cells have weakened, damaged or removed the functions of the endogenous genes of at least one encoding flocculating protein, including FLO5, FLO8 and FLO11.

The said yeast cells contain recombinant promoters operably attached to at least one gene encoding human albumin, where the promoter is preferably a genetically modified or not genetically modified inducible yeast AOX1 promoter.

The yeast cell contains at least one human superoxide dismutase or yeast superoxide dismutase encoding expression.

The yeast cell contains at least one human E3 ubiquitin ligase or yeast E3 ubiquitin ligase encoding expression.

### 2) expression cassette, that encodes at least one recombinant nucleic acid molecule of human albumin

For the said expression cassette, the nucleic acid constructs include but are not limited to the plasmids pHIL-D2, pPIC3.5, pHIL-S1, pPIC9, pPink-LC, pPink-HC, pPICZA, pPICZB, pPICZC, pPICZaA, pPICZaB and pPICZaC.

The recombinant promoter is operably linked to the signal peptide sequence, the encoding sequence of mating factor signal peptide of Saccharomyces cerevisiae (as shown in SEQ ID NO. 1) or the encoding sequence of signal peptide of human albumin (as shown in SEQ ID NO.2), and the encoding sequence of mature peptide of human albumin (as shown in SEQ ID NO.3).

### 3) expression cassette, that encodes at least one recombinant nucleic acid molecule that expresses superoxide dismutase

For the said expression cassette, the nucleic acid constructs include but are not limited to the plasmids pHIL-D2, pPIC3.5, pHIL-S1, pPIC9, pPink-LC, pPink-HC, pPICZA, pPICZB, pPICZC, pPICZaA, pPICZaB and pPICZaC.

The recombinant promoter is operably linked to the yeast superoxide dismutase encoding sequence. The encoding sequence of the yeast superoxide dismutase is as shown in SEQ ID NO.4.

The recombinant promoter is operably linked to the human superoxide dismutase encoding sequence. The encoding sequence of the human superoxide dismutase is as shown in SEQ ID NO.5.

### 4) expression cassette, that encodes at least one recombinant nucleic acid molecule that expresses E3 ubiquitin ligase

For the said expression cassette, the nucleic acid constructs include but are not limited to the plasmids pHIL-D2, pPIC3.5, pHIL-S1, pPIC9, pPink-LC, pPink-HC, pPICZA, pPICZB, pPICZC, pPICZaA, pPICZaB and pPICZaC.

The recombinant promoter is operably linked to the yeast E3 ubiquitin ligase encoding sequence. The encoding sequence of the yeast E3 ubiquitin ligase is as shown in SEQ ID NO.6.

According to but not limited to the method of this invention, culture the yeast cells under conditions suitable for the production of recombinant human albumin, induce the expression of exogenous protein and improve the expression of recombinant human albumin.

### Strains

E. coli DH5a component cells are used for all the E. coli cloning experiments.

The host cells of recombinant human albumin are yeast cells, which are preferred to be Pichia pastoris cells CBS7435, more preferably GS115 (Mut⁺ Pichia Pastoris strain with histidine dehydrogenase His4 gene mutation) and KM71 (Mut^{s} Pichia Pastoris strain with histidine dehydrogenase His4 gene mutation and AOX1 gene damage).

### Expression Vector

The expression vector needs to be integrated in the form of a single copy or multiple copies at the specific site of the host Pichia Pastoris cell genome, and achieve the homologous recombination with the chromosomes to allow the expression of the foreign gene. The pPic9 yeast expression vector (Invitrogen) and the pPicZA yeast expression vector (Invitrogen) are preferred.

### Reagents and Culture Media

StuI restriction endonuclease (NEB), PmeI restriction endonuclease (NEB), SacI restriction endonuclease (NEB), GoldVac EndoFree Plasmid Maxi Kit (ComWin Biotech), zeocin (Invitrogen), Geneticin G418 (Gibco), Ampicillin (Sangon Biotech (Shanghai) Co., Ltd.)

MD culture media, LB culture media, YPD culture media BMGY culture media and BMMY culture media

### Recombinant Plasmids

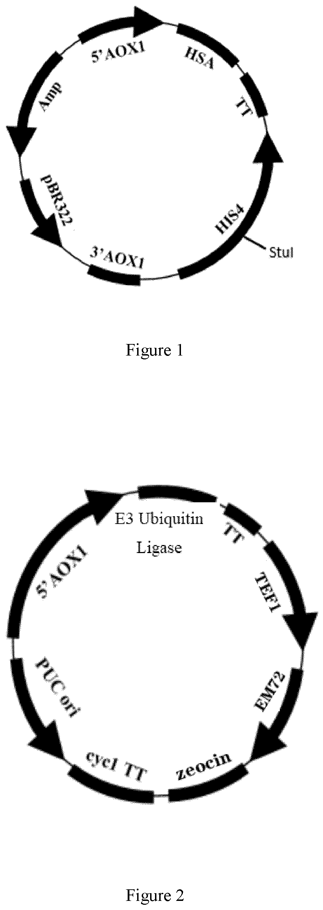

Recombinant human albumin plasmids, and the structure diagram is as shown in Figure 1.

Recombinant E3 ubiquitin ligase plasmids (yeast E3 ubiquitin ligase or human E3 ubiquitin ligase), and the structure diagrams are as shown in Figure 2.

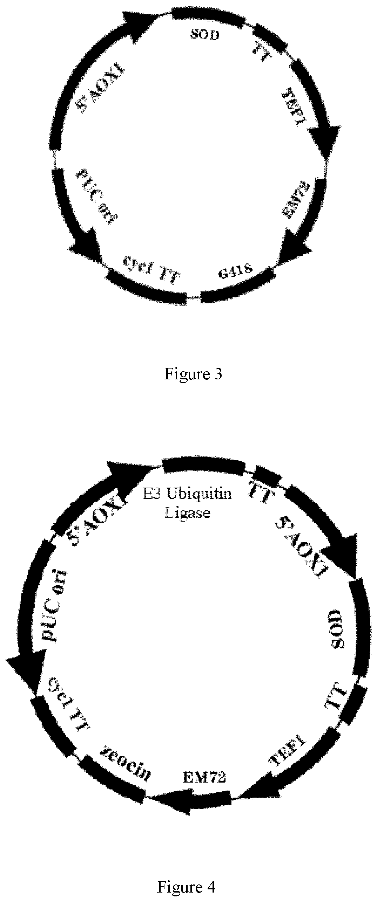

Recombinant superoxide dismutase plasmids (yeast superoxide dismutase or human superoxide dismutase), and the structure diagrams are as shown in Figure 3.

Recombinant superoxide dismutase (yeast E3 ubiquitin ligase or human E3 ubiquitin ligase) and E3 ubiquitin ligase (yeast superoxide dismutase or human superoxide dismutase) plasmid expression cassettes, and the structure diagrams are as shown in Figure 4.

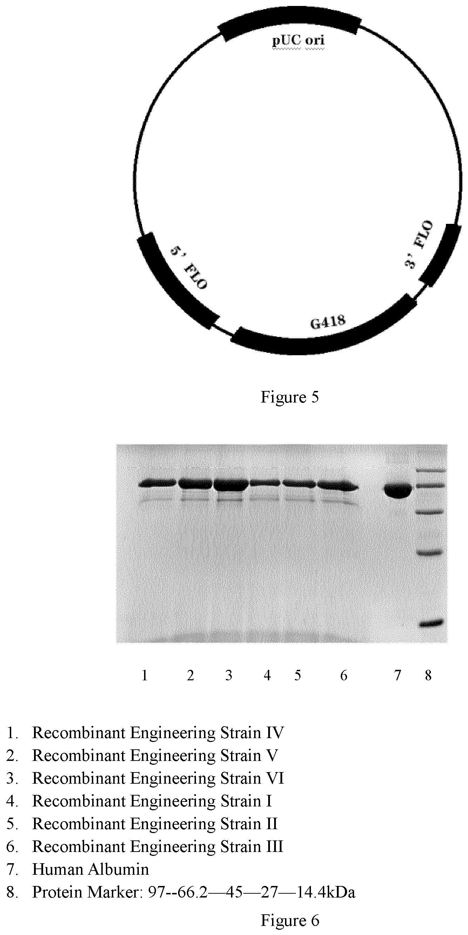

FLO gene knockout plasmid expression cassettes, and the structure diagrams are as shown in Figure 5.

### Transformation and Screening of Positive Clone

Transform the yeast cells using DNA restriction endonucleases PmeI, StuI, or SacI linear plasmids according to the electroporation method described in the operation manual of the Pichia yeast expression cassette (Invitrogen). Coat the cells on a selective plate with the appropriate antibiotics or nutritional deficiencies and incubate it at 30°C for 48 to 72 h.

Select the single colonies on the plate, place them into the culture media for incubation, and extract the genome DNA. Check the sequence table, and respectively use the HSA-F primer sequence (SEQ ID NO.10) and the HSA-R primer sequence (SEQ ID NO.11),
the yeast superoxide dismutase-F sequence (SEQ ID NO.12) and yeast superoxide dismutase-R sequence (SEQ ID NO.13), human superoxide dismutase-F sequence (SEQ ID NO.14) and human superoxide dismutase-R sequence (SEQ ID NO.15), yeast E3 ubiquitin ligase-F sequence (SEQ ID No.16) and yeast E3 ubiquitin ligase-R sequence (SEQ ID No.17) for PCR to screen the positive clones.

### Embodiment 1

This embodiment constructs the recombinant engineering strains that only contain the human albumin gene.

Recombinant engineering strain I: Transformed recombinant human albumin expression cassette, and the structure diagram is as shown in Figure 1. The encoding sequence of signal peptide is as shown in SEQ ID NO.1, and the encoding sequence of human albumin mature peptide is as shown in SEQ ID NO.3.

### Embodiment 2

This embodiment constructs an engineering strain that improves the expression of recombinant human albumin.

Recombinant engineering strain II: the recombinant engineering strain I is used as the host cell to transform the recombinant yeast E3 ubiquitin ligase expression cassette (the structure diagram is as shown Figure 2). The encoding sequence of the yeast E3 ubiquitin ligase is as shown in SEQ ID NO.6.

Recombinant engineering strain III: respectively transform the recombinant human albumin expression cassette and the FLO gene knockout plasmids. Construct the FLO gene knockout plasmids. The plasmid contains the homologous arm sequences on both sides of the FLO gene to be knocked out, and insert a selective G418 marker between the two homologous arms, and the structure diagram is as shown in Figure 5. The encoding sequence of yeast flocculating protein FLO5 is as shown in SEQ ID NO.7, the encoding sequence of yeast flocculating protein FLO8 is as shown in SEQ ID NO.8, and the encoding sequence of yeast flocculating protein FLO11 is as shown in SEQ ID NO.9.

Recombinant engineering strain IV: Respectively transform the recombinant human albumin expression cassette and the recombinant yeast superoxide dismutase expression cassette (the structure diagram is as shown in Figure 3). The encoding sequence of the yeast superoxide dismutase is as shown in SEQ ID NO.4.

Recombinant engineering strain V: the recombinant engineering strain III is used as the host cell to transform the recombinant yeast E3 ubiquitin ligase and recombinant yeast superoxide dismutase expression cassettes. The structure diagrams are as shown Figure 4.

Recombinant engineering strain VI: the recombinant engineering strain III is used as the host cell to transform the recombinant yeast E3 ubiquitin ligase and human superoxide dismutase expression cassettes. The structure diagrams are as shown Figure 4. The encoding sequence of the human superoxide dismutase is as shown in SEQ ID NO.5.

### Embodiment 3

Respectively take the recombinant engineering strains I, II, III, IV, V and VI, as shown in Table 1. Inoculate them in 40ml of BMGY culture solution at 220rpm and 30°C overnight, centrifuge the cell suspension, re-suspend the precipitate into 40ml of BMGY culture solution, start the induction at 220rpm and 30°C, add 0.4% methanol every 24 hours, centrifuge at 10,000rpm for 5 minutes after 96 hours, and collect the supernatant.

**Table 1**

| Recombinant Engineering Strain | Human Albumin Gene | Is the superoxide dismutase over expressed? | Is the E3 ubiquitin ligase over expressed? | Is the yeast flocculating protein knocked out? |
|---|---|---|---|---|
| I | Single Copy | No | No | No |
| II | Single Copy | No | Yes | No |
| III | Multiple Copies | No | No | Yes |
| IV | Multiple Copies | Yes | No | No |
| V | Multiple Copies | Yes | Yes | Yes |
| VI | Multiple Copies | Yes | Yes | Yes |

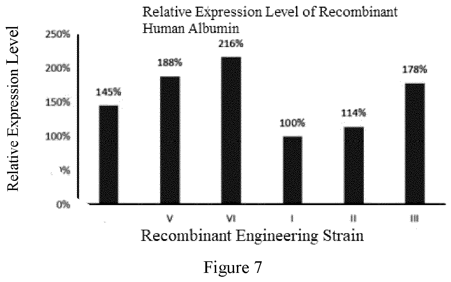

Perform the SDS-PAGE electrophoresis for the supernatant (see Figure 6 for the electrophoretograms), take the expression level of recombinant human albumin of recombinant engineering strain I as the control (100%), and it is noticed that the expression level of recombinant human albumin of each recombinant engineering strain is improved (see Figure 7 for the electrophoretograms). The relative expression level of recombinant human albumin of recombinant engineering strain VI is as high as 216%.

This invention involves a method for improving the expression of recombinant human albumin. In this specification, the term "recombinant human albumin" may also be referred to as "recombinant human serum albumin" and/or "recombinant human blood albumin" and/or "rHA" and/or "rHSA". The term "human serum albumin" refers to the human albumin extracted from human serum, which may also be referred to as "human blood albumin" and/or "HSA" and/or "HA" and/or "pdHSA".

It should be noted that this patent can realize the construction of a variety of strains, and the similar strains are also within the scope of protection of this patent.

At present, the most commonly used way to achieve large-scale production of recombinant microorganism expressed human albumin is mainly the yeast expression system. However, there can be premature flocculation during the growth and the metabolism of yeast cells, so precipitates from the fermentation broth can be resulted, which may lead to incomplete fermentation. When recombinant proteins are blocked at the disulfide bond formation phase or the transportation from the endoplasmic reticulum to the Golgi apparatus, the unfolded or misfolded proteins gather in the endoplasmic reticulum, causing a pressure in the endoplasmic reticulum and thus affecting the normal cell functions. Moreover, methanol metabolism will produce a large amount of reactive oxygen species, which will directly damage such cell components as nucleic acids, lipids and proteins, and even cause cell senescence or death in severe cases. According to but not limited to the method of this invention, culture the yeast cells under conditions suitable for the production of recombinant human albumin, induce the expression of protein and significantly improve the expression of recombinant human albumin.

The above shows and describes the basic principle, main features and advantages of this invention. The individuals skilled in the art should be able to understand that this invention is not limited by the above embodiments, the above embodiments and the description only describe the principle of this invention. Without deviating from the spirit and scope of this invention, this invention is subject to various changes and improvements, and these changes and improvements fall within the scope of protection of the claims.

## Claims

1. A method for improving the expression of recombinant human albumin, which is **characterized by** that,
The host cell of the method, while expressing recombinant human albumin, also:
Weakens, damages or removes the functions of the endogenous genes of at least one encoding flocculating protein;
And/or, edits the endogenous genes or the heterologous genes to allow the over-expression of the superoxide dismutase;
And/or, edits the endogenous genes or the heterologous genes to allow the overexpression of the E3 ubiquitin ligase.

2. The method as described in Claim 1 for improving the expression of recombinant human albumin, which is **characterized by** that the host cell is a kind of yeast cell.

3. The method as described in Claim 1 for improving the expression of recombinant human albumin, which is **characterized by** that the endogenous genes of the encoding flocculating protein include FLO5, FLO8 and FLO11.

4. The method as described in Claim 1 for improving the expression of recombinant human albumin, which is **characterized by** that the endogenous genes or the heterologous genes encoding superoxide dismutase are genetically modified or not genetically modified; the endogenous genes or the heterologous genes encoding E3 ubiquitin ligase are genetically modified or not genetically modified.

5. The method as described in Claim 1 for improving the expression of recombinant human albumin, which is **characterized by** that the gene encoding superoxide dismutase is yeast superoxide dismutase gene; or the gene encoding superoxide dismutase is human copper-zinc superoxide dismutase nucleic acid molecular gene.

6. The method as described in Claim 1 for improving the expression of recombinant human albumin, which is **characterized by** that the gene encoding E3 ubiquitin ligase is yeast E3 ubiquitin ligase; or the gene encoding E3 ubiquitin ligase is human E3 ubiquitin ligase nucleic acid molecule.

7. The method as described in Claim 2 for improving the expression of recombinant human albumin, which is **characterized by** that the yeast cell is the cell of one or more of the following yeasts: Hansenula, Pichia and Candida.

8. The method as described in Claim 7 for improving the expression of recombinant human albumin, which is **characterized by** that the Pichia cell is Pichia pastoris cell.

9. The method as described in Claim 1 for improving the expression of recombinant human albumin, which is **characterized by** that the recombinant expression cassette of the gene encoding human albumin, the gene encoding superoxide dismutase, and the gene encoding E3 ubiquitin ligase is based on a nucleic acid construct or the nucleic acid construct of different selection markers.

10. The method as described in Claim 9 for improving the expression of recombinant human albumin, which is **characterized by** that the nucleic acid constructs include any or any combination of the plasmids pHIL-D2, pPIC3.5, pHIL-S1, pPIC9, pPink-LC, pPink-HC, pPICZA, pPICZB, pPICZC, pPICZaA, pPICZaB and pPICZaC; the recombinant promoters include any or any combination of the AOX1 promoter, the GAP promoter, the GAL promoter, the FDH promoter and the FLD promoter.

11. The method as described in Claim 10 for improving the expression of recombinant human albumin, which is **characterized by** that there is at least one recombinant promoter modified human albumin encoded in the host cell; there is at least one recombinant promoter modified superoxide dismutase encoded in the host cell; or there is at least one recombinant promoter modified E3 ubiquitin ligase encoded in the host cell.

12. The method as described in any of the Claims 1 to 11 for improving the expression of recombinant human albumin, which is **characterized by** that the sequence used is any or any combination of the following sequences:
SEQ ID NO.1 encoding sequence of mating factor signal peptide of Saccharomyces cerevisiae;
SEQ ID NO.2 encoding sequence of signal peptide of human albumin;
SEQ ID NO.3 encoding sequence of mature peptide of human albumin;
SEQ ID NO.4 encoding sequence of yeast superoxide dismutase;
SEQ ID NO.5 encoding sequence of human superoxide dismutase;
SEQ ID NO.6 encoding sequence of yeast E3 ubiquitin;
SEQ ID NO.7 encoding sequence of yeast flocculating protein FLO5;
SEQ ID NO.8 encoding sequence of yeast flocculating protein FLO5;
SEQ ID NO.9 encoding sequence of yeast flocculating protein FLO11;
SEQ ID NO.10 HSA-F primer sequence;
SEQ ID NO.11 HSA-R primer sequence;
SEQ ID NO.12 sequence of yeast superoxide dismutase-F;
SEQ ID NO. 13 sequence of yeast superoxide dismutase-R;
SEQ ID NO.14 sequence of human superoxide dismutase-F;
SEQ ID NO.15 sequence of human superoxide dismutase-R;
SEQ ID NO.16 encoding sequence of yeast E3 ubiquitin-F;
SEQ ID NO.17 encoding sequence of yeast E3 ubiquitin-R;

13. The method as described in any of the Claims 1 to 12 for improving the expression of recombinant human albumin, which is **characterized by** that, recombinant engineering strain I: the transformed recombinant human albumin expression cassette, the signal peptide encoding sequence is as shown in SEQ ID NO.1, and the human albumin mature peptide encoding sequence is as shown in SEQ ID NO.3.
Recombinant engineering strain II: the recombinant engineering strain I is used as the host cell to transform the recombinant yeast E3 ubiquitin ligase expression cassette; and the E3 ubiquitin ligase encoding sequence is as shown in SEQ ID NO.6.
Recombinant engineering strain III: respectively transform recombinant human albumin expression cassette and FLO gene knockout plasmids; construct FLO gene knockout plasmids, there are homologous arm sequences of the two sides of the FLO gene to be knocked out on the plasmids, a selective G418 marker is inserted between the two homologous arm sequences; the yeast flocculating protein FLO5 encoding sequence is as shown in SEQ ID NO.7, the yeast flocculating protein FLO8 encoding sequence is as shown in SEQ ID NO.8, and the yeast flocculating protein FLO11 encoding sequence is as shown in SEQ ID NO.9.
Recombinant engineering strain IV: respectively transform recombinant human albumin expression cassette and recombinant yeast superoxide dismutase expression cassette; and the yeast superoxide dismutase encoding sequence is as shown in SEQ ID NO.4.
Recombinant engineering strain V: the recombinant engineering strain III is used as the host cell to transform the recombinant yeast E3 ubiquitin ligase and recombinant yeast superoxide dismutase expression cassettes.
Recombinant engineering strain VI: the recombinant engineering strain III is used as the host cell to transform the recombinant yeast E3 ubiquitin ligase and human superoxide dismutase expression cassettes; and the human superoxide dismutase encoding sequence is as shown in SEQ ID NO.5.
Respectively take the recombinant engineering strains I, II, III, IV, V and VI, as shown in Table 1; Inoculate them in 40ml of BMGY culture solution at 220rpm and 30°C overnight, centrifuge the cell suspension, re-suspend the precipitate into 40ml of BMGY culture solution, start the induction at 220rpm and 30°C, add 0.4% methanol every 24 hours, centrifuge at 10,000rpm for 5 minutes after 96 hours, and collect the supernatant.
**Table 1**
| Recombinant Engineering Strain | Human Albumin Gene | Is the superoxide dismutase over expressed? | Is the E3 ubiquitin ligase over expressed? | Is the yeast flocculating protein knocked out? |
|---|---|---|---|---|
| I | Single Copy | No | No | No |
| II | Single Copy | No | Yes | No |
| III | Multiple Copies | No | No | Yes |
| IV | Multiple Copies | Yes | No | No |
| V | Multiple Copies | Yes | Yes | Yes |
| VI | Multiple Copies | Yes | Yes | Yes |
Perform the SDS-PAGE electrophoresis for the supernatant, take the expression level of recombinant human albumin of recombinant engineering strain I as the control (100%), and it is noticed that the expression level of recombinant human albumin of each recombinant engineering strain is improved. The relative expression level of recombinant human albumin of recombinant engineering strain VI is as high as 216%.

14. The host cells manipulated by any or any combination of the sequence genes SEQ ID NO.1 - SEQ ID NO.17.

15. The recombinant albumins expressed by host cells manipulated by any or any combination of the sequence genes SEQ ID NO.1 - SEQ ID NO.17.
